# EUROPÄISCHE PATENTSCHRIFT

(11) **EP 0 687 280 B1**
(45) Veröffentlichungstag und Bekanntmachung des Hinweises auf die Patenterteilung: **04.06.1997**
(21) Anmeldenummer: 94909908.9
(22) Anmeldetag: 02.03.1994
(51) Int. Cl.: C08G 18/38, C08G 18/10, C08G 18/48, A61K 6/10

(54) **KUNSTSTOFFE MIT EINEM GEHALT AN SILAN-, ÄTHER-, URETHAN- UND HARNSTOFFGRUPPEN UND IHRE VERWENDUNG ALS DENTALMASSEN**
PLASTIC MATERIALS WITH A SILANE, ETHER, URETHANE AND UREA GROUP CONTENT AND THEIR USE AS DENTAL COMPOSITIONS
MATIERES PLASTIQUES CONTENANT DES GROUPES SILANE, ETHER, URETHANNE ET UREE ET LEUR UTILISATION COMME COMPOSITIONS DENTAIRES

(30) Priorität: 05.03.1993 DE 4307024
(43) Veröffentlichungstag der Anmeldung: 20.12.1995
(73) Patentinhaber: ERNST MÜHLBAUER KG, D-22547 Hamburg (DE)
(72) Erfinder: LÜBBERS, Dierk, D-22523 Hamburg (DE); MÜHLBAUER, Wolfgang, D-22605 Hamburg (DE)
(74) Vertreter: Glawe, Delfs, Moll & Partner
(86) Internationale Anmeldenummer: EP9400607
(87) Internationale Veröffentlichungsnummer: WO9420560

(56) Entgegenhaltungen:
- EP-A- 0 170 865
- EP-A- 0 410 199
- DE-A- 3 636 974

## Beschreibung

Die Erfindung betrifft Kunststoffe mit mindestens einem Silan-, Ether-, Urethan- und Harnstoffgruppen enthaltenden Polyadditionsprodukt mit einer überwiegend linearen Molekülstruktur mit ausschließlich aliphatisch oder cycloaliphatisch gebundenen Ether-, Urethan- und Harnstoffsegmenten und einem Zahlenmittel der Molmasse im Bereich von 800 bis 20.000, wobei das Polyadditionsprodukt die folgenden Merkmale aufweist:
a) einen Gehalt an Polyethergruppen von 25 bis 90, insbesondere 50 bis 80 Gew.-Teilen, auf 100 Gew.-Teile Polyadditionsprodukt;
b) einen Gehalt an Urethangruppen der Formel I

   - HN - CO - O - (I)

   von 0,5 bis 10, insbesondere von 1 bis 8 Gew.-Teilen auf 100 Gew.-Teile Polyadditionsprodukt;
c) einen Gehalt an Harnstoffgruppen der Formel II

   - NH - CO - NH - (II)

   von 0,5 bis 10, insbesondere von 1 bis 8 Gew.-Teilen auf 100 Gew.-Teile Polyadditionsprodukt;
d) einen Gehalt an beiden Enden der überwiegend linearen Molekülstruktur angeordneten Alkoxysilylgruppen der Formel III

   - NR - (CH₂)ₘ - SiR¹R²R³ (III)

   in der
   - m: die Zahl 3,
   - R: Wasserstoff oder eine Gruppe der Formel (IV)

   - (CH₂)ₘ - SiR¹R²R³ (IV)

   mit den hier angegebenen Bedeutungen für m, R¹, R² und R³, und
   mindestens eine der Gruppen R¹, R² und R³ eine Gruppe der Formel V

      - (O - CₚH₂ₚ)_{q} - O - A - R⁴ (V)
in der
- p: die Zahl 3 und
- q: eine Zahl im Bereich von 1 bis 100, insbesondere von 2 bis 4 sowie
- A: eine Einfachbindung und
- R⁴: eine Alkyl-, Aralkyl-, Vinyl-, Vinylcarbonyl, alpha-Methylvinylcarbonyl- oder beta-Methylvinylcarbonylgruppe ist,
bedeuten,
wobei die restlichen Gruppen R¹, R² und R³ Methyl, Ethyl oder C₁- bis C₄-Alkoxy bedeuten, soweit sie nicht Gruppen der obigen Definitionen sind,
und die Kunststoffe weiterhin mindestens einen Katalysator für die Kondensation der Silangruppen enthalten.

Weitere vorteilhafte Ausgestaltungen der Erfindung ergeben sich aus der folgenden Beschreibung und den Unteransprüchen.

Die Kunststoffe der Erfindung eignen sich insbesondere als Abform- und Modelliermassen, insbesondere für Dentalzwecke und den Formenbau, mit geringer Schrumpfungsneigung nach dem Aushärten.

Kunststoffe auf Basis von Silan-, Ether-, Urethan- und Harnstoffgruppen enthaltenden Polyadditionsprodukten in Mischung mit polymerisierbaren Verbindungen sind aus der EP-A 0 170 865 unter der EP-A 0 410 199 bekannt; die Polyadditionsprodukte als solche sind in der DE-A 36 36 974 beschrieben; auf den Inhalt dieser Schriften wird Bezug genommen.

Die in den vorbekannten Kunststoffen bzw. in den darin enthaltenen Polyadditionsprodukten auftretenden Alkoxysilylgruppen weisen eine Struktur gemäß der obigen Formel III auf, wobei mindestens eine der Gruppen R¹, R² und R³ C₁-C₄-Alkoxy, bevorzugt Methoxy oder Ethoxy, bedeutet und R² und R³ die gleiche Bedeutung wie R¹ aufweisen können oder Methyl- oder Ethylgruppen bedeuten.

Es ist bevorzugt, wenn in den Silylgruppen der Formel III eine oder zwei der Gruppen R¹, R² und R³ Methyl, Ethyl oder Methoxy bedeuten.

Infolge der enthaltenen Alkoxysilangruppen sind diese Verbindungen in Gegenwart geeigneter saurer Katalysatoren zur Kondensation befähigt, wobei als gewissermaßen erste Stufe der Härtung elastische, gelartige Polymere entstehen. Diese Polymere können anschließend in Abhängigkeit der Art der zugesetzten polymerisierbaren Olefine zu formbeständigen, starren Materialien nachgehärtet werden. Speziell die Härtung in der ersten Stufe führt jedoch zu bei Abform- bzw. Modelliermassen unerwünschten Schrumpfungsvorgängen, da die bei der Kondensation gebildeten Kondensationsprodukte mit dem Polymermaterial unverträglich sind und aus den erhaltenen Formkörpern unter Schrumpfung derselben austreten. So wird z.B. in der DE-A 36 36 974 für eines der dort erwähnten Systeme für die Dimensionsänderung des in der ersten Stufe gehärteten Formkörpers ein Wert von 2,2% nach 120 min angegeben; ein derartiges Material ist insbesondere für solche Dentalzwecke, bei denen es auf höchste Formgenauigkeit ankommt, nicht brauchbar.

Für die aus der EP 0 170 865 bekannten Kunststoffe sind überhaupt keine Angaben über das Schrumpfungsverhalten beim Aushärten und über ihre Eignung für Dentalzwecke offenbart worden; zudem sind diese Kunststoffe insofern mit gewissen Nachteilen behaftet, als sie infolge ihres Gehaltes an Ethylenoxygruppen hydrophil sind und in Anwesenheit von Wasser quellen können.

Die vorliegende Erfindung ist auf Kunststoffe der oben genannten Art gerichtet, bei denen nach dem Härten keine bzw. gegenüber dem Stand der Technik erheblich verringerte Schrumpfungstendenzen auftreten und die bei Wasserzutritt nicht quellen. Die Erfindung beruht auf der Erkenntnis, daß die Schrumpfungsneigungen vermieden bzw. herabgesetzt werden können, wenn man die im Verlauf der Härtung freigesetzten Kondensationsprodukte so einstellt, daß sie mit den als Grundgerüst vorhandenen Polyadditionsprodukten so verträglich wie möglich sind und daher bei ihnen nicht die Tendenz besteht, daß sie aus dem Polymerkörper unter Schrumpfung desselben austreten bzw. sich an der Oberfläche desselben ansammeln, womit sie für dentale Werkstoffe besonders geeignet sind. Diese Aufgabe wird durch die eingangs erwähnten Kunststoffe der Erfindung gelöst.

Die den Kunststoffe der Erfindung zugrundeliegenden Polyadditionsprodukte können hergestellt werden, indem man aliphatische oder cycloaliphatische Diisocyanate bzw. Mischungen derselben mit linearen Polyethern mit endständigen freien Hydroxylgruppen mit einem Zahlenmittel der Molmassen im Bereich von 250 bis 6000 umsetzt, wobei man gegebenenfalls zusätzlich noch aliphatische oder cycloaliphatische Alkandiole bzw. Mischungen derselben mit einem Zahlenmittel der Molmassen im Bereich von 62 bis weniger als 300 zusetzen kann. Die erhaltenen Prepolymeren werden üblicherweise mit Alkoxysilylmonoaminen umgesetzt; dabei kann man gegebenenfalls noch aliphatische und/oder cycloaliphatische Diamine mit primären Aminogruppen mit einem Zahlenmittel der Molmassen von 60 bis 300 mitverwenden. Die gegebenenfalls eingesetzten Diamine dienen zur Einstellung des jeweils gewünschten Molekulargewichts.

Hierfür geeignete Diisocyanate sind insbesondere aliphatische oder cycloaliphatische Diisocyanate, bei denen die Diisocyanatgruppen an aliphatische Kohlenwasserstoffreste mit 2 bis 12 Kohlenstoffatomen oder cycloaliphatische bzw. gemischt aliphatisch-cycloaliphatische Kohlenwasserstoffreste mit 4 bis 15 Kohlenstoffatomen gebunden sind.

Typische Beipiele für geeignete Diisocyanate sind Ethylendiisocyanat, Tetramethylendiisocyanat, Hexamethylendiisocyanat, Dodecamethylendiisocyanat, Cyclobutan-1,3-diisocyanat, Cyclohexan-1,3- und 1,4-diisocyanat oder 1-Isocyanat-3,3,5-trimethyl-5-isocyanatomethyl-cyclohexan bzw. Isophorondiisocyanat. Es können auch Gemische der vorgenannten Diisocyanate eingesetzt werden. Besonders bevorzugt ist Isophorondiisocyanat.

Im Rahmen der Erfindung einsetzbare Polyetherdiole können insbesondere durch random-Polymerisation oder Blockpolymerisation von Epoxiden wie Ethylenoxid, Propylenoxid, Butylenoxid, Tetrahydrofuran oder Epichlorhydrin erhalten werden, oder auch durch Anlagerung dieser Epoxide, gegebenenfalls im Gemisch oder nacheinander, an Startkomponenten mit reaktionsfähigen Wasserstoffatomen wie Alkohole oder Amine, bzw. Wasser, Ethylglykol oder 1,2-Propylenglykol. Bevorzugt werden solche Polyether eingesetzt, deren freie OH-Gruppen überwiegend primäre OH-Gruppen sind.

Im Rahmen der Erfindung geeignete Diamine sind z.B. primäre Aminogruppen aufweisende, aliphatische, cycloaliphatische oder gemischt aliphatisch-cycloaliphatische Diamine mit einem Zahlenmittel der Molmassen im Bereich von 60 bis 300. Typische Beispiele sind Ethylendiamin, Tetramethylendiamin, Hexamethylendiamin, 4,4'-Diaminodicyclohexylmethan, 1,4-Diaminocyclohexan, 4,4'-Diamino-3,3'-dimethyl-dicyclohexyimethan oder 1-Amino-3,3,5-trimethyl-5-aminomethyl-cyclohexan(Isophorondiamin). Besonders bevorzugt sind 4,4'-Diaminodicyclohexylmethan und Isophorondiamin.

Typische Beispiele für Alkandiole sind Ethylenglykol, 1,2-Propylenglykol, 1,4-Butylenglykol, 2,3-Butylenglykol, 1,6-Hexandiol, 1,8-Octandiol, Neopentylglykol, Cyclohexandimethanol, 1,4-bis-Hydroxymethylcyclohexan, 2-Methyl-1,3-propandiol, 3-Methylpentan-1,5-diol, Diethylenglykol, Triethylenglykol, Tetraethylenglykol, Polyethylenglykole, Dipropylenglykol, Tri- und Tetrapropylenglykol, Polypropylenglykol, Dibutylenglykol und Polybutylenglykol.

Geeignete Alkoxysilylamine für die Einführung von Alkoxysilylgruppen der allgemeinen Formel III in die Kunststoffe der Erfindung können hergestellt werden, indem man z.B. aus der DE-A 36 36 974 bekannte Alkoxysilanverbindungen, insbesondere die im Handel erhältlichen gamma-Aminopropyl-tri-C₁-C₄-alkoxysilane bzw. bis-(3-C₁-C₄-Alkoxysilylpropyl)-amine, bevorzugt gamma-Aminopropyl-trimethoxy- oder -triethoxysilan, einer Umesterung mit Monohydroxyverbindungen der allgemeinen Formel VI

H - (O - CₚH₂ₚ)_{q} - O - A - R⁴ (VI)

mit den obigen Bedeutungen für p, q, A und R⁴ unterwirft.

R⁴ kann weiterhin polymerisierbare olefinische Doppelbindungen aufweisen; so strukturierte Kunststoffe weisen dann Polykondensations- neben Polymerisationszentren auf und sind zweistufig härtbar. Im ersten Fall ist von einer großen Verträglichkeit der bei der Kondensation austretenden Gruppe VI mit dem "backbone"- Material auszugehen. In dem anderen Fall erfolgt bei der nachfolgenden Polymerisation, z.B. einer Licht-, Redox- oder Heißpolymerisation, der freigesetzten Verbindung (VI) eine Fixierung derselben in dem "backbone"-Material. Daraus folgen die günstigen Schrumpfeigenschaften der Kunststoffe der Erfindung.

Den Polyadditionsprodukten der Erfindung können, soweit sie nicht bereits polymerisierbare olefinische Doppelbindungen enthalten, jedoch auch dann, die üblichen, radikalisch härtbaren Monomeren zugesetzt werden, insbesondere an sich bekannte, monofunktionelle oder polyfunktionelle (Meth)acrylate, insbesondere Methylmethacrylat, Isobutylmethacrylat, Cyclohexylmethacrylat, Ethylenglykoldimethacrylat, Diethylenglykoldimethacrylat, Polyethylenglykoldimethacrylat, Butandioldimethacrylat, Hexandioldimethacrylat, Decandioldimethacrylat, Dodecandioldimethacrylat, Bisphenol-A-dimethacrylat, Trimethylolpropantrimethacrylat, weiterhin Bis-GMA sowie Reaktionsprodukte aus Isocyanaten, inbesondere Di- und/oder Triisocyanate und OH-Gruppen-haltige Methacrylate. Typische Beipiele für die zuletzt genannten Verbindungen sind Umsetzungsprodukte von 1 Mol Hexamethylendiisocyanat mit 2 Mol 2-Hydroxyethylmethacrylat, von 1 Mol tris-(6-Isocyanatohexyl)isocyanurat mit 3 Mol Hydroxyethylmethacrylat und von 1 Mol Trimethylhexamethylendiisocyanat mit 2 Mol Hydroxyethylmethacrylat. Der Anteil dieser Verbindungen in der Mischung mit dem bzw. den Silikopolyethern kann zwischen 5 und 90 Gew.-% variieren.

Geeignete Katalysatoren für die Heißpolymerisation sind Peroxide wie Dibenzoylperoxid, Dilaurylperoxid, tert.-Butylperoctoat oder tert.-Butylcarbenzoat, weiterhin auch alpha, alpha-Azo-bis-(isobutyroethylester)-benzpinakol und 2,2'-Dimethylbenzpinakol.

Als Katalysatoren für die Lichtpolymerisation sind die üblichen Photosensibilisatoren zusammen mit einem Reduktionsmittel bevorzugt, z.B. alpha-Diketone wie 9,10-Phenanthrenchinon, Diacetyl, Furil, Anisil, 4,4'-Dichlorbenzil und 4,4'-Dialkoxybenzil. Besonders bevorzugt ist Campherchinon. Beispiele für Reduktionsmittel sind Amine wie Cyanoethylmethylanilin, Dimethylaminoethylmethacrylat, n-Butylamin, Triethylamin, Triethanolamin, N,N-Dimethylanilin oder N-Methyldiphenylamin.

Typische Beipiele für sogenannte Redoxpolymerisationskatalysatoren sind Peroxide in Mischung mit einem Reduktionsmittel, z.B. auf Basis tertiärer aromatischer Amine und Dibenzoylperoxid, Dilaurylperoxid und Di-4-chlorbenzoylperoxid.

Beispiele für Reagentien zum Vernetzen von Epoxygruppen sind Polyamine oder Dicarbonsäureanhydride.

Die Polykondensation an den Silangruppierungen können durch anorganische und/oder organische Säuren als Katalysatoren gefördert werden; typische Beipiele für diese Katalysatoren sind z.B. saure Ionenaustauscher, Phosphorsäure, Dibutylphosphorsäure, verdünnte Schwefelsäure, Weinsäure, Zitronensäure, Adipinsäure, Salzsäure, Sulfonsäure und dergleichen; Salzsäure und Phosphorsäure sind besonders bevorzugt.

Den Kunststoffen der Erfindung können weiterhin anorganische oder organische Füllstoffe zugesetzt werden, die an sich bekannt sind. Geeignete Füllstoffe sind insbesondere Quarz, Aluminiumoxid, Aluminiumhydroxid, Kieselgel, Aerosil^{R}, Metalloxid, Gips, Korund, Glas, Polymerperlen, Zeolithe; reaktive Feststoffe wie silanisierte bzw. oberflächenbehandelte inerte Feststoffe, Silikate, Zementpulver; weiterhin Oligomere oder Polymere auf Basis der hier angesprochenen Polyadditionsprodukte; sowie Parafine, Wachse, Vaseline, Duft- und Aromastoffe. Den Kunststoffen der Erfindung können weiterhin auch Farbstoffe, gegebenenfalls auch solche, die bei erfolgter Aushärtung einen Farbumschlag ergeben, zugesetzt werden, weiterhin auch Emulgatoren zur Einstellung des rheologischen Verhaltens, Antibiotika, blutstillende Mittel und dergleichen. Der Füllstoffanteil in dem Kunststoff der Erfindung kann im allgemeinen im Bereich von 40 bis 80 Gew.-% liegen.

Die Erfindung wird im folgenden anhand bevorzugter Ausführungsbeispiele sowie von Vergleichsbeispiele näher erläutert.

### Vergleichsbeispiel 1:

a) 912,5 g (0,5 Mol OH) eines linearen Polyetherdiols (Molekulargewicht 3650, Blockcopolymer hergestellt durch Polyaddition von 80 Gew.-Teilen Propylenoxid auf Propylenglykol und anschließender Polyaddition von 30 Gew.-Teilen Ethylenoxid) wurden 1 Stunde lang bei 100°C im Ölpumpenvakuum (0,2 hPa) getrocknet. Nach Abkühlen auf Raumtemperatur wurde dem Ansatz 111 g (0,5 Mol) Isophorondiisocyanat und 1 Tropfen Zinnethylhexanoat hinzugefügt. Unter einem leichten Stickstoffstrom wurde der Ansatz auf 100°C geheizt und 60 min bei dieser Temperatur belassen. Nach Abkühlen auf Raumtemperatur wurde die NCO-Zahl des Prepolymeren bestimmt:
   NCO gef.: 1,87%
   NCO ber.: 2,05%
   Dann wurden 89,65 g (0,5 Mol) 3-Aminopropyltrimethoxysilan innerhalb von 2 Stunden zugetropft, so daß es zu einer nur mäßigen Erwärmung des Reaktionsgemisches kam. Nach einer weiteren Stunde Rühren bei Raumtemperatur, war im IR-Spektrum keine NCO-Bande mehr zu erkennen.
   Der entstandene Poly(ether-urethan-harnstoff) ist eine klare, nur wenig gelblich gefärbte und gut fließfähige Masse.
b) Zur Vernetzung der in der vorstehenden Stufe a) hergestellten Prepolymeren wird eine Härtersubstanz, bestehend aus 15 Gew.-% Phosphorsäure, 35 Gew.-% Wasser (bidestilliert) und 50 Gew.-% Glycerin, angerührt.
   20 g Prepolymer aus Stufe a) werden mit 0,7 g der vorgenannten Härterflüssigkeit 30 sek lang auf dem Mischblock verrührt.
   Die Masse härtet innerhalb weniger Minuten zu einem elastischen, nicht klebrigen Formkörper aus.

Die Dimensionsänderung, bestimmt gemäß ISO 4823 (Trockenlagerung, 23°C), beträgt:

| | |
|---|---|
| 30 Minuten | 0,1% |
| 60 Minuten | 0,2% |
| 120 Minuten | 0,3% |
| 12 Stunden | 1,0% |
| 24 Stunden | 1,1% |

### Beispiel 1:

a) Man verfährt wie im Vergleichsbeispiel 1, Stufe a), beschrieben, nur daß als Aminosilanverbindung 221,8 g (0,5 Mol) 3-Aminopropyl-tris-(2-methoxy-ethoxy-ethoxy)-silan zugetropft wird. Der erhaltene Poly(ether-urethan-harnstoff) ist ebenfalls gut fließfähig, klar und leicht gelblich gefärbt.
b) 20 g Prepolymer aus Stufe a) werden mit 0,6 g der unter Vergleichs-beispiel 1, Stufe b) beschriebenen Härterflüssigkeit 30 sek lang auf dem Mischblock verrührt. Die Masse härtet innerhalb einiger Minuten zu einem gummieleastischen, nichtklebrigen Körper aus.

Die Dimensionsänderung (Trockenlagerung, 23°C, ISO 4823) beträgt:

| | |
|---|---|
| 30 Minuten | 0% |
| 60 Minuten | 0% |
| 120 Minuten | 0% |
| 12 Stunden | 0,5% |
| 24 Stunden | 1,1% |

### Beispiel 2:

a) 89,65 g (0,5 Mol) 3-Aminopropyltrimethoxysilan und 525 g (1,5 Mol) Polyethylenglykol-monomethylether (MG 350) werden mit 0,1 g Natrium zunächst 3 Stunden bis zur Auflösung des Natriums bei 80°C gerührt. Dann wird der Ansatz auf 150°C erhitzt und das aus der Umesterungsreaktion entstehende Methanol abdestilliert und aufgefangen. Nach 12 Stunden wird der Ansatz auf 70°C abgekühlt und für 5 Stunden Ölpumpenvakuum (2 hPa) angelegt. Dann wird der Ansatz im Stickstoffstrom abgekühlt.
b) Man tropft 582,65g des vorstehend in Stufe a) erhaltenen Umesterungsprodukts zu dem im Vergleichsbeispiel 1, Stufe a) aus 912,5g linearen Polyetherdiol und 111g Isophorondiisocyanat erhaltenen Prepolymer. Infrarotspektroskopisch sind dann keine Isocyanatgruppen mehr nachzuweisen.
c) Anschließend wird eine Härterpaste, bestehend aus 33 Gew.-% Phosphorsäure, 17 Gew.-% Wasser (bidestilliert) und 50 Gew.-% Glycerin, angemischt.

20 g der Substanz aus Stufe b) und 0,93 g der Härterpaste werden auf dem Mischblock 30 sek gemischt. Die Masse härtet innerhalb von einigen Minuten zu einem gummielastischen, nichtklebrigen Festkörper aus.

Die physikalischen Eigenschaften wurden gemäß ISO 4823 bestimmt. Die Dimensionsänderung beträgt:

| | |
|---|---|
| nach 30 Minuten | 0,0% |
| nach 60 Minuten | 0,0% |
| nach 2 Stunden | 0,0% |
| nach 5 Stunden | 0,17% |
| nach 24 Stunden | 0,8% |

### Beispiel 3:

a) 44,82 g (0,25 mol) 3-Aminopropyltrimethoxysilan und 525 g (0,75 mol Polypropylenglykol-monobutylether (MG = 700)) werden mit 0,1 g Natrium zunächst 3 Stunden bis zur Auflösung des Natriums bei 80°C gerührt. Dann wird der Ansatz auf 150°C erhitzt und das aus der Umesterungsreaktion entstehende Methanol abdestilliert und aufgefangen. Nach 12 Stunden wird der Ansatz auf 70°C abgekühlt und für 5 Stunden Ölpumpenvakuum (2 mbar) angelegt. Dann wird der Ansatz im Stickstoffstrom abgekühlt.
b) Man verfährt wie in Vergleichsbeispiel 1 beschrieben. Als lineares Polyetherdiol werden jedoch 500 g (0,25 mol OH) Polypropylenglykol (Molekulargewicht 4000) und entsprechend 55,57 g (0,25 mol) Isophorondiisocyanat eingesetzt. Als Aminosilanreaktionspartner werden 553,75 g des Umesterungsproduktes aus Stufe a) innerhalb von 2 Stunden zugetropft. Infrarotspektroskopisch sind dann keine Isocyanatgruppen mehr nachzuweisen.
c) Es wird eine Härterpaste bestehend aus 42,5 Gew.-% Phosphorsäure, 7,5 Gew.-% Wasser (bidestilliert) und 50 Gew.-% Glycerin angemischt.

20 g der Substanz aus Stufe b) und 1,63 g der Härterpaste werden auf dem Mischblock 30 Sekunden gemischt. Die Masse härtet innerhalb von einigen Minuten zu einem gummielastischen, nichtklebrigen Festkörper aus.

Die Dimensionsänderung, bestimmt gemäß ISO 4823, Trockenlagerung, 23°C, beträgt:

| | |
|---|---|
| nach 30 Minuten | 0,0% |
| nach 60 Minuten | 0,0% |
| nach 2 Stunden | 0,2% |
| nach 5 Stunden | 0,2% |
| nach 24 Stunden | 0,4%. |

### Beispiel 4:

a) Umesterung
   17,93g (0,1 mol) 3-Aminopropyltrimethoxysilan und 123,77g (0,6 mol) Tripropylenglykolmonomethylether werden unter Feuchtigkeitsausschluß zunächst mit 0,lg Natrium 2 Stunden bei 60°C gerührt. Nach Auflösen des Natriums wird die Temperatur innerhalb von 4 Stunden auf 150°C erhöht und der bei der Umesterungsreaktion entstehende Methanol abdestilliert und aufgefangen. Nach 12 Stunden wird bei dieser Temperatur für 5 Stunden Ölpumpenvakuum angelegt. Dabei destillieren 55,76g (0,27 mol) überschüssiger Tripropylenglykolmonomethylether ab.
   n = 1,4295 (Lit.: 1,4300)
   Die Bestimmung des Amingehalts des im Destillationssumpf verbliebenen Umesterungsproduktes zeigt, daß ein quantitativer Austauch der Methoxygruppen stattgefunden hat.
   - Amingehalt:: Gef.: 2,26%
   - Ber.:: 2,28% 3-Aminopropyl-tris-(2-methoxy-tripropoxy)silan
b) Darstellung des Prepolymeren
   Man verfährt wie in Vergleichsbeispiel 1 beschrieben. Als lineares Polyetherdiol werden jedoch 40g (8 mmol) Polypropylenglykol (Molekulargewicht 4000) und entsprechend 3,56g (16 mmol) Isophorondiisocyanat eingesetzt. Als Aminosilanreaktionspartner werden 11,23g (16 mmol) des Umesterungsproduktes aus Beispiel 9 zugetropft. Nach 4 Stunden Rühren bei Raumtemperatur ist infrarotspektroskopisch kein Isocyanat mehr nachzuweisen.
c) Aushärtung
   Es wird eine Härterpaste bestehend aus 50 Gew.% Glycerin, 12 Gew.% Salzsäure und 38 % Gew.% Wasser angemischt.

20g der Substanz aus Stufe b) werden mit 0,53%g dieser Härterpaste auf dem Mischblock 30 s gemischt. Innerhalb von einigen Minuten härtet diese Masse zu einem nichtklebrigen, gummielastischen Körper aus.
Die Dimensionsänderung, bestimmt gemäß ISO 4823 (23°C, Trokkenlagerung) beträgt:

| | |
|---|---|
| nach 30 Minuten | 0,0% |
| nach 60 Minuten | 0,0% |
| nach 2 Stunden | 0,0% |
| nach 5 Stunden | 0,0% |
| nach 24 Stunden | 0,1%. |

## Patentansprüche

1. Kunststoffe mit mindestens einem Silan-, Ether-, Urethan- und Harnstoffgruppen enthaltenden Polyadditionsprodukt.mit einer überwiegend linearen Molekülstruktur mit ausschließlich aliphatisch oder cycloaliphatisch gebundenen Ether-, Urethan- und Harnstoffsegmenten und einem Zahlenmittel der Molmasse im Bereich von 800 bis 20.000, wobei das Polyadditionsprodukt die folgenden Merkmale aufweist:
a) einen Gehalt an Polyethergruppen von 25 bis 90, insbesondere 50 bis 80 Gew.-Teilen, auf 100 Gew.-Teile Polyadditionsprodukt;
b) einen Gehalt an Urethangruppen der Formel I
- HN - CO - O - (I)
von 0,5 bis 10, insbesondere von 1 bis 8 Gew.-Teilen auf 100 Gew.-Teile Polyadditionsprodukt;
c) einen Gehalt an Harnstoffgruppen der Formel II
- NH - CO - NH - (II)
von 0,5 bis 10, insbesondere von 1 bis 8 Gew.-Teilen auf 100 Gew.-Teile Polyadditionsprodukt;
d) einen Gehalt an beiden Enden der überwiegend linearen Molekülstruktur angeordneten Alkoxysilylgruppen der Formel III
- NR - (CH₂)ₘ - SiR¹R²R³ (III)
in der
m die Zahl 3,
R Wasserstoff oder eine Gruppe der Formel (IV)
-(CH₂)ₘ - SiR¹R²R³ (IV)
mit den hier angegebenen Bedeutungen für m, R¹, R² und R³, und
mindestens eine der Gruppen R¹, R² und R³ eine Gruppe der Formel V
- (O - CₚH₂ₚ)_{q} - O - A - R⁴ (V)
in der
p die Zahl 3, und
q eine Zahl im Bereich von 1 bis 100, insbesondere von 2 bis 4 sowie
A eine Einfachbindung und
R⁴ eine Alkyl-, Aralkyl-, Vinyl-, Vinylcarbonyl, alpha-Methylvinylcarbonyl- oder beta-Methylvinylcarbonylgruppe ist,
bedeuten,
wobei die restlichen Gruppen R¹, R² und R³ Methyl, Ethyl oder C₁- bis C₄-Alkoxy bedeuten, soweit sie nicht Gruppen der obigen Definitionen sind,
und die Kunststoffe weiterhin mindestens einen Katalysator für die Kondensation der Silangruppen enthalten.

2. Kunststoffe nach Anspruch 1, dadurch gekennzeichnet, daß in den Silylgruppen der Formel III eine oder zwei der Gruppen R¹, R² und R³ Methyl, Ethyl oder Methoxy bedeuten.

3. Kunststoff nach Anspruch 1 oder 2 im Gemisch mit mindestens einem radikalisch härtbaren, mono- oder polyfunktionellen (Meth)acrylat sowie mindestens einem Katalysator für die Heiß-, Kalt- oder Lichtpolymerisation.

4. Verwendung von Kunststoffen mit mindestens einem Silan-, Ether-, Urethan- und Harnstoffgruppen enthaltenden Polyadditionsprodukt mit einer überwiegend linearen Molekülstruktur mit ausschließlich aliphatisch oder cycloaliphatisch gebundenen Ether-, Urethan- und Harnstoffsegmenten und einem Zahlenmittel der Molmasse im Bereich von 800 bis 20.000, wobei das Polyadditionsprodukt die folgenden Merkmale aufweist:
a) einen Gehalt an Polyethergruppen von 25 bis 90, insbesondere 50 bis 80 Gew.-Teilen, auf 100 Gew.-Teile Polyadditionsprodukt;
b) einen Gehalt an Urethangruppen der Formel I
- HN - CO - O - (I)
von 0,5 bis 10, insbesondere von 1 bis 8 Gew.-Teilen auf 100 Gew.-Teile Polyadditionsprodukt;
c) einen Gehalt an Harnstoffgruppen der Formel II
- NH - CO - NH - (II)
von 0,5 bis 10, insbesondere von 1 bis 8 Gew.-Teilen auf 100 Gew.-Teile Polyadditionsprodukt;
d) einen Gehalt an beiden Enden der überwiegend linearen Molekülstruktur angeordneten Alkoxy-Silylgruppen der Formel III
- NR - (CH₂)ₘ - SiR¹R²R³ (III)
in der
m die Zahl 3,
R Wasserstoff oder eine Gruppe der Formel (IV)
-(CH₂)ₘ - SiR¹R²R³ (IV)
mit den hier angegebenen Bedeutungen für m, R¹, R² und R³, und
mindestens eine der Gruppen R¹, R² und R³ eine Gruppe der Formel V
- (O - CₚH₂ₚ)_{q} - O - A - R⁴ (V)
in der
p die Zahl im Bereich von 2 bis 4, insbesondere von 2 bis 3, und
q eine Zahl im Bereich von 1 bis 100, insbesondere von 2 bis 4 sowie
A eine Einfachbindung und
R⁴ eine Alkyl-, Aralkyl-, Vinyl-, Vinylcarbonyl, alpha-Methylvinylcarbonyl- oder beta-Methylvinylcarbonylgruppe ist,
bedeuten,
wobei die restlichen Gruppen R¹, R² und R³ Methyl, Ethyl
oder C₁- bis C₄-Alkoxy bedeuten, soweit sie nicht Gruppen der obigen Definitionen sind,
und die Kunststoffe weiterhin mindestens einen Katalysator für die Kondensation der Silangruppen enthalten, als Abform-, Dublier- und Modelliermassen.

5. Verwendung nach Anspruch 4, dadurch gekennzeichnet, daß in den Silylgruppen der Formel III eine oder zwei der Gruppen R¹, R² und R³ Methyl, Ethyl oder Methoxy bedeuten.

6. Verwendung nach Anspruch 4 oder 5 zusammen mit mindestens einem radikalisch härtbaren, mono- oder polyfunktionellen (Meth)acrylat sowie mindestens einem Katalysator für die Heiß-, Kalt- oder Lichtpolymerisation als mehrstufig härtende Polymermassen.

## Claims

1. Plastics with at least one polyaddition product which contains silane, ether, urethane and urea groups and has a predominantly linear molecular structure with exclusively aliphatically or cycloaliphatically bonded ether, urethane and urea segments and a number-average molecular weight in the range from 800 to 20,000, the polyaddition product having the following features:
a) a content of polyether groups of 25 to 90, in particular 50 to 80 parts by weight per 100 parts by weight of polyaddition product;
b) a content of urethane groups of the formula I
-HN-CO-O- (I)
of 0.5 to 10, in particular 1 to 8 parts by weight per 100 parts by weight of polyaddition product;
c) a content of urea groups of the formula II
-NH-CO-NH- (II)
of 0.5 to 10, in particular 1 to 8 parts by weight per 100 parts by weight of polyaddition product;
d) a content of alkoxysilyl groups, located on both ends of the predominantly linear molecular structure, of the formula III
-NR-(CH₂)ₘ-SiR¹R²R³ (III)
in which
m is the number 3,
R is hydrogen or a group of the formula (IV)
-(CH₂)ₘ-SiR¹R²R³ (IV)
with the meanings given here for m, R¹, R² and R³, and at least one of the groups R¹, R² and R³ is a group of the formula V
-(O-CₚH₂ₚ)_{q}-O-A-R⁴ (V)
in which
p is the number 3 and
q is a number in the range from 1 to 100, in particular from 2 to 4, and
A is a single bond and
R⁴ is an alkyl, aralkyl, vinyl, vinylcarbonyl, alphamethylvinylcarbonyl or beta-methylvinylcarbonyl group, and in which the remaining groups R¹, R² and R³ are methyl, ethyl or C₁- to C₄-alkoxy, if they are not groups of the above definitions,
and the plastics furthermore comprising at least one catalyst for condensation of the silane groups.

2. Plastics according to Claim 1, characterized in that, in the silyl groups of the formula III, one or two of the groups R¹, R² and R³ are methyl, ethyl or methoxy.

3. Plastics according to Claim 1 or 2 mixed with at least one mono- or polyfunctional (meth)acrylate which can be cured by means of free radicals and at least one catalyst for hot polymerization, cold polymerization or photopolymerization.

4. Use of plastics with at least one polyaddition product which contains silane, ether, urethane and urea groups and has a predominantly linear molecular structure with exclusively aliphatically or cycloaliphatically bonded ether, urethane and urea segments and a number-average molecular weight in the range from 800 to 20,000, the polyaddition product having the following features:
a) a content of polyether groups of 25 to 90, in particular 50 to 80 parts by weight per 100 parts by weight of polyaddition product;
b) a content of urethane groups of the formula I
-HN-CO-O- (I)
of 0.5 to 10, in particular 1 to 8 parts by weight per 100 parts by weight of polyaddition product;
c) a content of urea groups of the formula II
-NH-CO-NH- (II)
of 0.5 to 10, in particular 1 to 8 parts by weight per 100 parts by weight of polyaddition product;
d) a content of alkoxysilyl groups, located on both ends of the predominantly linear molecular structure, of the formula III
-NR-(CH₂)ₘ-SiR¹R²R³ (III)
in which
m is the number 3,
R is hydrogen or a group of the formula (IV)
-(CH₂)ₘ-SiR¹R²R³ (IV)
with the meanings given here for m, R¹, R² and R³, and at least one of the groups R¹, R² and R³ is a group of the formula V
-(O-CₚH₂ₚ)_{q}-O-A-R⁴ (V)
in which
p is a number in the range from 2 to 4, in particular from 2 to 3 and
q is a number in the range from 1 to 100, in particular from 2 to 4, and
A is a single bond and
R⁴ is an alkyl, aralkyl, vinyl, vinylcarbonyl, alphamethylvinylcarbonyl or beta-methylvinylcarbonyl group, and in which the remaining groups R¹, R² and R³ are methyl, ethyl or C₁- to C₄-alkoxy, if they are not groups of the above definitions,
and the plastics furthermore comprising at least one catalyst for condensation of the silane groups, as impression, duplicating and modelling compositions.

5. Use according to Claim 4, characterized in that in the silyl groups of the formula III, one or two of the groups R¹, R² and R³ are methyl, ethyl or methoxy.

6. Use according to Claim 4 or 5 together with at least one mono- or polyfunctional (meth)acrylate which can be cured by means of free radicals and at least one catalyst for hot polymerization, cold polymerization or photopolymerization, as polymer compositions which cure in several stages.

## Revendications

1. Matières plastiques comportant au moins un produit de polyaddition contenant des groupes silane, éther, uréthane et urée, avec une structure moléculaire principalement linéaire relié avec des segments éther, uréthane et urée exclusivement aliphatiques ou cycloaliphatiques, et une valeur de la masse molaire comprise entre 800 et 20 000, le produit de polyaddition présentant les caractéristiques suivantes :
a) une teneur en groupe polyéther de 25 à 90, en particulier de 50 à 80, parties en poids, pour 100 parties en poids de produit de polyaddition;
b) une teneur en groupes uréthane de formule I
- HN - CO - O - (I)
de 0,5 à 10, en particulier de 1 à 8 parties en poids, pour 100 parties en poids de produit de polyaddition;
c) une teneur en groupes urée de formule II
- NH - CO - NH - (II)
de 0,5 à 10, en particulier de 1 à 8 parties en poids, pour 100 parties en poids de produit de polyaddition;
d) une teneur en groupes alcoxysilyle de formule III, placés aux deux extrémités de la structure moléculaire principalement linéaire
- NR - (CH₂)ₘ - SiR¹R²R³ (III)
dans laquelle
m représente le nombre 3,
R, un atome d'hydrogène ou un groupe de formule (IV)
-(CH₂)ₘ - SiR¹R²R³ (IV)
avec les significations indiquées ici pour m, R¹, R² et R³, et
au moins un des groupes R¹, R² et R³ représente un groupe de formule V
-(O - CₚH₂ₚ)_{q} - O - A - R⁴ (V)
dans laquelle
p représente le nombre 3, et
q, un nombre dans la plage comprise entre 1 et 100, en particulier entre 2 et 4, et
A une liaison simple, et
R⁴ est un groupe alkyle, aralkyle, vinyle, vinylcarbonyle, alpha-méthylvinylcarbonyle ou beta-méthylvinylcarbonyle,
les groupes R¹, R² et R³ restants représentant alors des groupes méthyle, éthyle ou alcoxy en C₁ à C₄, dans la mesure où ils ne sont pas des groupes répondant aux définitions ci-dessus,
et les matières plastiques contiennent en outre au moins un catalyseur pour la condensation des groupes silane.

2. Matières plastiques selon la revendication 1, caractérisées en ce que, dans les groupes silyle de formule III, un ou deux des groupes R¹, R² et R³ représentent un groupe méthyle, éthyle ou méthoxy.

3. Matières plastiques selon la revendication 1 ou 2, en mélange avec au moins un (méth)acrylate mono- ou polyfonctionnel, durcissable, radicalaire, ainsi qu'au moins un catalyseur pour la polymérisation à chaud, à froid ou à la lumière.

4. Utilisation de matières plastiques comportant au moins un produit de polyaddition contenant des groupes silane, uréthane, éther et urée, avec une structure moléculaire principalement linéaire relié avec des segments éther, uréthane et urée exclusivement aliphatiques ou cycloaliphatiques, et une valeur de la masse molaire comprise entre 800 et 20 000, le produit de polyaddition présentant les caractéristiques suivantes :
a) une teneur en groupes polyéther de 25 à 90, en particulier de 50 à 80, parties en poids, pour 100 parties en poids de produit de polyaddition;
b) une teneur en groupes uréthane de formule I
- HN - CO - O - (I)
de 0,5 à 10, en particulier de 1 à 8 parties en poids, pour 100 parties en poids de produit de polyaddition;
c) une teneur en groupes urée de formule II
- NH - CO - NH - (II)
de 0,5 à 10, en particulier de 1 à 8 parties en poids, pour 100 parties en poids de produit de polyaddition;
d) une teneur en groupes alcoxysilyle de formule III, placés aux deux extrémités de la structure moléculaire principalement linéaire
- NR - (CH₂)ₘ - SiR¹R²R³ (III)
dans laquelle
m représente le nombre 3,
R, un atome d'hydrogène ou un groupe de formule (IV)
-(CH₂)ₘ - SiR¹R²R³ (IV)
avec les significations indiquées ici pour m, R¹, R² et R³, et
au moins un des groupes R¹, R² et R³ représente un groupe de formule V
-(O - CₚH₂ₚ)_{q} - O - A - R⁴ (V)
dans laquelle
p représente un nombre compris entre 2 et 4, en particulier entre 2 et 3, et
q, un nombre dans la plage comprise entre 1 et 100, en particulier entre 2 et 4, et
A une liaison simple, et
R⁴ est un groupe alkyle, aralkyle, vinyle, vinylcarbonyle, alpha-méthylvinylcarbonyle ou beta-méthylvinylcarbonyle,
les groupes R¹, R² et R³ restants représentant alors des groupes méthyle, éthyle ou alcoxy en C₁ à C₄, dans la mesure où ils ne sont pas des groupes répondant aux définitions ci-dessus,
et les matières plastiques contiennent en outre au moins un catalyseur pour la condensation des groupes silane sous forme de masses à mouler, à doubler et à modeler.

5. Utilisation selon la revendication 4, caractérisée en ce que, dans les groupes silyle de formule III, un ou deux des groupes R¹, R² et R³, représentent un groupe méthyle, éthyle ou méthoxy.

6. Utilisation selon la revendication 4 ou 5, en association avec au moins un (méth)acrylate mono- ou polyfonctionnel, durcissable, radicalaire, ainsi qu'avec au moins un catalyseur pour la polymérisation à chaud, à froid ou à la lumière, sous forme de masses polymères durcissant en plusieurs étapes.
